# EUROPÄISCHE PATENTANMELDUNG

(11) **EP 4 039 172 A1**
(43) Veröffentlichungstag der Anmeldung: **10.08.2022**
(21) Anmeldenummer: 21155035.5
(22) Anmeldetag: 03.02.2021
(51) Int. Cl.: A61B 5/00, A61B 5/01, A61B 5/145

(54) **DENTALES SENSORSYSTEM ZUR BEFESTIGUNG EINES DENTALSENSORS**

(71) Anmelder: Ivoclar Vivadent AG, 9494 Schaan (LI)
(72) Erfinder: Krybus, Robert Wolfgang, 9479 Oberschan (CH); Moosmann, Florian, 6835 Muntlix (AT)
(74) Vertreter: Baldus, Oliver

(57) **Zusammenfassung**

Die vorliegende Erfindung betrifft ein dentales Sensorsystem (100), mit einer ersten Zahnbefestigungsvorrichtung (101-1) zum Befestigen an einem ersten Zahn (103-1); einer zweiten Zahnbefestigungsvorrichtung (101-2) zum Befestigen an einem zweiten Zahn (103-2); einer Sensorschiene (105) zwischen der ersten und der zweiten Zahnbefestigungsvorrichtung (101-1; 101-2); und einem Dentalsensor (107), der entlang der Sensorschiene (105) positionierbar ist.

## Beschreibung

Die vorliegende Erfindung betrifft ein dentales Sensorsystem zur Befestigung eines Dentalsensors, eine dentale Sensorbefestigungsvorrichtung, einen Dentalsensor und ein Verfahren zum Positionieren eines Dentalsensors.

Gegenwärtig sind unterschiedliche intraorale Befestigungs- der Anbringungsmöglichkeiten von Dentalsensoren bekannt. Beispielsweise existieren Molarbänder, die eine Messvorrichtung für die Bestimmung eines pH-Wertes aufnehmen. Diese werden an einem Molar mittels einer Zementierung befestigt. Zudem sind Kronen bekannt, die intergierte Messvorrichtungen umfassen. In diesem Fall muss jedoch die Krone für einen Austausch des Dentalsensors vom Zahn entfernt werden. Des Weiteren existieren Ansätze, dass bei von einem Implantat getragenen Zähnen die Messvorrichtung in einem Abutment angebracht ist. In diesen Vorrichtungen kann der Dentalsensor einerseits jedoch nicht leicht ausgewechselt werden. Andererseits kann die Messung nur an einer Stelle durchgeführt werden.

Es ist die technische Aufgabe der vorliegenden Erfindung, eine flexible Positionierung eines Dentalsensors innerhalb einer Mundhöhle zu ermöglichen.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein dentales Sensorsystem gelöst, mit einer ersten Zahnbefestigungsvorrichtung zum Befestigen an einem ersten Zahn; einer zweiten Zahnbefestigungsvorrichtung zum Befestigen an einem zweiten Zahn; einer Sensorschiene zwischen der ersten und der zweiten Zahnbefestigungsvorrichtung; und mindestens einem Dentalsensor, der entlang der Sensorschiene positionierbar ist. Dadurch wird der technische Vorteil erreicht, dass der mindestens eine Dentalsensor an einer beliebigen Stelle der Sensorschiene angeordnet werden kann. Mittels der Sensorschiene ist die Position des mindestens einen Dentalsensors frei wählbar und nach Bedarf anpassbar. Es wird eine abstandsgetreue und wiederholbare Messung erreicht. Der mindestens eine Dentalsensor kann beispielsweise manuell oder mit einer Zunge verschoben werden.

In einer technisch vorteilhaften Ausführungsform des dentalen Sensorsystems sind die erste und/oder zweite Zahnbefestigungsvorrichtung durch einen Zahnaufsatz gebildet. Dadurch wird beispielsweise der technische Vorteil erreicht, dass das dentale Sensorsystem auf die Zähne aufsteckbar ist und einen guten Halt findet.

In einer weiteren technisch vorteilhaften Ausführungsform des dentalen Sensorsystems ist der mindestens eine Dentalsensor entlang der Sensorschiene verschiebbar. Dadurch wird beispielsweise der technische Vorteil erreicht, dass der Dentalsensor auch nach einer anfänglichen Positionierung entlang der Sensorschiene an unterschiedliche Positionen bewegt werden kann, ohne dass der Dentalsensor von der Sensorschiene genommen werden muss.

In einer weiteren technisch vorteilhaften Ausführungsform des dentalen Sensorsystems ist die Sensorschiene an der ersten und/oder der zweiten Zahnbefestigungsvorrichtung befestigbar. Dadurch wird beispielsweise der technische Vorteil erreicht, dass die Sensorschiene nachträglich an den Zahnbefestigungsvorrichtungen angebracht werden kann.

In einer weiteren technisch vorteilhaften Ausführungsform des dentalen Sensorsystems umfasst die erste und/oder zweite Zahnbefestigungsvorrichtung einen Kugelkopf zum Befestigen der Sensorschiene. Dadurch wird beispielsweise der technische Vorteil erreicht, dass sich die Sensorschiene zusätzlich drehen und räumlich einrichten lässt.

In einer weiteren technisch vorteilhaften Ausführungsform des dentalen Sensorsystems ist der Dentalsensor auf/in die Sensorschiene aufsetzbar oder aufklickbar. Dadurch wird beispielsweise der technische Vorteil erreicht, dass eine einfache und schnelle Befestigung des Dentalsensors erreicht wird.

In einer weiteren technisch vorteilhaften Ausführungsform des dentalen Sensorsystems umfasst die Sensorschiene einen ersten Nutabschnitt und/oder einen zweiten Nutabschnitt zum Führen des Dentalsensors. Dadurch wird beispielsweise der technische Vorteil erreicht, dass der Dentalsensor auf einfache Weise entlang der Sensorschiene geführt werden kann.

In einer weiteren technisch vorteilhaften Ausführungsform des dentalen Sensorsystems umfasst der Dentalsensor einen elastischen Streifen zum Einsetzen zwischen dem ersten und/oder dem zweiten Nutabschnitt. Dadurch wird beispielsweise der technische Vorteil erreicht, dass die Lage des Dentalsensors zusätzlich stabilisiert wird.

In einer weiteren technisch vorteilhaften Ausführungsform des dentalen Sensorsystems umfasst die Sensorschiene eine Durchgangsöffnung zum Gebiss oder Zahnfleisch hin. Dadurch wird beispielsweise der technische Vorteil erreicht, dass der Dentalsensor Messungen direkt im Bereich des Gebisses oder Zahnfleischs vorgenommen werden können.

In einer weiteren technisch vorteilhaften Ausführungsform des dentalen Sensorsystems umfasst die Sensorschiene ein Zahnprofil zum Eingreifen des Dentalsensors. Dadurch wird beispielsweise der technische Vorteil erreicht, dass ein Verrutschen oder unbeabsichtigtes Bewegen des Dentalsensors verhindert wird.

In einer weiteren technisch vorteilhaften Ausführungsform des dentalen Sensorsystems umfasst der Dentalsensor eine Antriebsvorrichtung zum Bewegen des Dentalsensors entlang der Sensorschiene. Dadurch wird beispielsweise der technische Vorteil erreicht, dass der Dentalsensor selbständig unterschiedliche Positionen an der Sensorschiene anfahren kann.

In einer weiteren technisch vorteilhaften Ausführungsform des dentalen Sensorsystems umfasst das Sensorsystem eine Spannungsversorgungsvorrichtung und/oder eine Datenübermittlungsvorrichtung für den Dentalsensor. Die Datenübermittlungsvorrichtung kann beispielsweise durch eine Bluetooth-Schnittstelle oder eine NFC-Schnittstelle (NFC - Near Field Communication) gebildet sein, damit erfasste Daten von dem Dentalsensor nach außen übertragen werden können und extern ausgewertet werden können. Dadurch wird beispielsweise der technische Vorteil erreicht, dass der Dentalsensor entlang der Sensorschiene mit elektrischer Leistung versorgt werden und Daten extern ausgewertet werden können.

Gemäß einem zweiten Aspekt wird die technische Aufgabe durch eine dentale Sensorbefestigungsvorrichtung gelöst, mit einer ersten Zahnbefestigungsvorrichtung zum Befestigen an einem ersten Zahn; einer zweiten Zahnbefestigungsvorrichtung zum Befestigen an einem zweiten Zahn; und einer Sensorschiene zwischen der ersten und der zweiten Zahnbefestigungsvorrichtung, auf der ein Dentalsensor positionierbar ist. Dadurch werden die gleichen technischen Vorteile, wie durch das dentale Sensorsystem nach dem ersten Aspekt erreicht.

Gemäß einem dritten Aspekt wird die technische Aufgabe durch einen Dentalsensor für eine dentale Sensorbefestigungsvorrichtung nach dem zweiten Aspekt gelöst. Dadurch werden die gleichen technischen Vorteile, wie durch das dentale Sensorsystem nach dem ersten Aspekt erreicht.

Gemäß einem vierten Aspekt wird die technische Aufgabe durch ein Verfahren zum Positionieren eines Dentalsensors gelöst, mit den Schritten eines Befestigens einer dentalen Sensorbefestigungsvorrichtung mit einer ersten Zahnbefestigungsvorrichtung an einem ersten Zahn und einer zweiten Zahnbefestigungsvorrichtung an einem zweiten Zahn; und eines Positionierens eines Dentalsensors an einer Sensorschiene zwischen der ersten Zahnbefestigungsvorrichtung und der zweiten Zahnbefestigungsvorrichtung. Dadurch werden die gleichen technischen Vorteile, wie durch das dentale Sensorsystem nach dem ersten Aspekt erreicht.

Gemäß einem fünften Aspekt wird die technische Aufgabe durch Verwenden einer Sensorschiene zwischen einer ersten Zahnbefestigungsvorrichtung und einer zweiten Zahnbefestigungsvorrichtung zum Positionieren eines Dentalsensors gelöst. Dadurch werden die gleichen technischen Vorteile, wie durch das dentale Sensorsystem nach dem ersten Aspekt erreicht.

Ausführungsbeispiele der Erfindung sind in den Zeichnungen dargestellt und werden im Folgenden näher beschrieben.

Es zeigen:
- Fig. 1: eine schematische Ansicht eines dentalen Sensorsystems;
- Fig. 2: eine weitere schematische Ansicht des dentalen Sensorsystems;
- Fig. 3: eine weitere schematische Ansicht des dentalen Sensorsystems;
- Fig. 4: unterschiedliche Ausführungsformen einer Sensorschiene; und
- Fig. 5: ein Blockdiagramm eines Verfahrens zum Positionieren eines Dentalsensors.

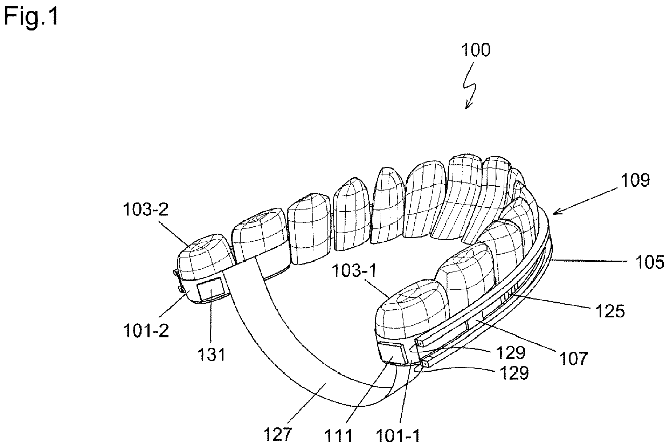

Fig. 1 zeigt eine schematische Ansicht eines dentalen Sensorsystems 100. Das dentale Sensorsystem 100 umfasst eine erste Zahnbefestigungsvorrichtung 101-1 zum Befestigen an einem oder mehreren ersten Zähnen 103-1, eine zweite Zahnbefestigungsvorrichtung 101-2 zum Befestigen an einem oder mehreren zweiten Zähnen 103-2, eine Sensorschiene 105 und einen oder mehrere Dentalsensoren 107.

Die Zahnbefestigungsvorrichtungen 101-1 und 101-2 sind beispielsweise durch entsprechende geformte Kunststoffkappen als Zahnaufsatz gebildet, die auf die jeweiligen Zähne aufsetzbar sind. Die Kunststoffkappen sind beispielsweise durch individuell gedruckte Molaraufsätze gebildet, die mittels eines additiven oder subtraktiven Verfahrens hergestellt worden sind. Die Zahnbefestigungsvorrichtungen 101-1 und 101-2 können auch ausgebildet sein, um an einem Kleber, einem Abutment, einem Implantataufsatz und/oder einer Spanngenschiene befestigt zu werden.

Die Zahnbefestigungsvorrichtungen 101-1 und 101-2 sind optional über einen Bügel 127 miteinander verbunden.

Zwischen den Zahnbefestigungsvorrichtungen 101-1 und 101-2 ist eine Sensorschiene 105 angeordnet, auf der ein oder mehrere Dentalsensoren 107 positionierbar sind und auswechselbar befestigt sind. Die Sensorschiene 105 wird für den individuellen Zahnbogen bereitgestellt. Die Zahnbefestigungsvorrichtungen 101-1 und 101-2 und/oder die Sensorschiene 105 kann individuell additiv oder subtraktiv aus einem geeigneten Material hergestellt werden, wie beispielsweise Kunststoff. Die Sensorschiene 105 kann aus einem elastischen Material hergestellt sein, so dass sich diese an den Zahnbogen anpasst und leicht beweglich bleibt. In der Sensorschiene 105 können ein der mehrere Dentalsensoren 107 verschiebbar geführt werden. In der Sensorschiene 105 können allerdings auch einzelne Steckplätze mit Klickelementen vorgesehen sein, um die Dentalsensoren 107 an der Sensorschiene 105 zu befestigen. Wenn mehrere Dentalsensoren 107 verwendet werden, kann für jeden Dentalsensor 107 ein Bereich mit Sperren oder mit Segmenten auf der Sensorschiene 105 eingerichtet werden. Die Sensorschiene 105 kann auch ein Zahnprofil 125 umfassen, das entlang der Sensorschiene 105 verläuft. An dem Zahnprofil 125 können die einzelnen Dentalsensoren 125 einrasten. Daher kann das Zahnprofil 125 verwendet werden, um die Dentalsensoren 107 in der Position festzulegen. Das Zahnprofil 125 kann auch als Eingriff für ein entsprechendes Zahnrad einer Antriebsvorrichtung dienen, um den Dentalsensor 107 entlang der Sensorschiene 105 fortzubewegen.

Die Sensorschiene 105 kann über den gesamten Zahnbogen, oberhalb oder unterhalb eines Zahnbogens verlaufen, damit die Zahnbereiche frei zugänglich sind. Zusätzlich kann die Sensorschiene auch das Zahnfleisch mit umfassen oder nur am Zahnfleisch anliegen. Die Anordnung der Sensorschiene 105 kann einzeln oder zusammen mit einer weiteren Sensorschiene 105 im labialen oder lingualen Bereich erfolgen.

Die Zahnbefestigungsvorrichtungen 101-1 und 101-2 und die Sensorschiene 105 bilden zusammen eine dentale Sensorbefestigungsvorrichtung 109. Eine Spannungsversorgungsvorrichtung 111, wie beispielsweise eine Batterie, kann in der Zahnbefestigungsvorrichtung 101-1 und 101-2 oder dem Bügel 127 vorgesehen oder integriert sein. Die elektrische Spannung, die von der Spannungsversorgungsvorrichtung 111 bereitgestellt wird, kann über zwei elektrische Leiter 129 an den jeweiligen Dentalsensor 107 geführt werden. Die elektrischen Leiter 129 laufen getrennt entlang der Sensorschiene 105. Dadurch kann der Dentalsensor 107 an jeder Stelle mit elektrischer Leistung versorgt werden.

Eine Datenübermittlungsvorrichtung 131 kann ebenfalls im Bügel 127 oder in den Zahnbefestigungsvorrichtungen 101-1 und 101-2 vorgesehen sein. Durch die Datenübermittlungsvorrichtung 131 lassen sich die Daten, die durch den Dentalsensor 107 erfasst worden sind, drahtlos nach außen senden. Daneben können Steuerdaten für den jeweiligen Dentalsensor 107 empfangen werden. Zu diesem Zweck umfasst die Datenübermittlungsvorrichtung 131 eine geeignete elektronische Schaltung.

Der Dentalsensor 107 ist entlang der Sensorschiene 105 verschiebbar. Der Dentalsensor 107 kann beispielsweise ein Sensor sein, der zum Messen eines pH-Wertes, einer EthanolKonzentration, einer Lactat-Konzentration, einer Cortisol-Konzentration, einer Glucose-Konzentration, einer Ionenkonzentration, zum Messen von Schallwellen beim Aufeinanderbeißen und/oder ein Sensor zum Messen einer Temperatur. Zu diesem Zweck umfasst der Dentalsensor 107 geeignete elektronische Schaltungen, die auf flexiblen Leiterplatten mit einer eigenen Spannungsversorgung angeordnet sein können.

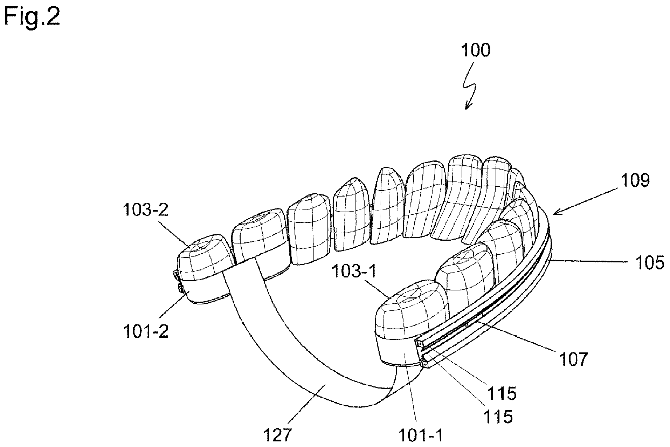

Fig. 2 zeigt eine weitere schematische Ansicht des dentalen Sensorsystems 100. Die Sensorschiene 105 kann nach innen ragende Dichtlippen 115 umfassen, die dazu dienen, den Flüssigkeitskontakt zu den elektrischen Leitern 129 zu verhindern, die innerhalb der Nutabschnitte verlaufen. Die Dichtlippen 115 erstecken sich ebenfalls entlang der Sensorschiene 105 und sind aus einem elastischen Material gebildet. Die Dichtlippen 115 sind an der Außenseite der Nutabschnitte angeordnet und ragen diagonal in den Zwischenraum zwischen den beiden Nutabschnitten hinein.

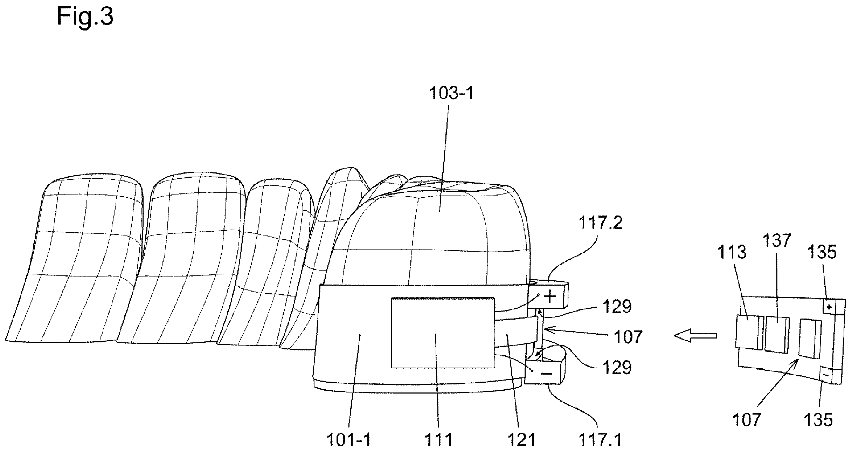

Fig. 3 zeigt eine weitere schematische Ansicht des dentalen Sensorsystems 100. Die Sensorschiene 105 weist einen ersten Nutabschnitt 117-1 und einen zweiten gegenüberliegenden Nutabschnitt 117-2 zum Führen und Halten des Dentalsensors 107 auf. Der Dentalsensor 107 wird zwischen den beiden Nutabschnitten 117-1 und 117-2 gehalten und in Längsrichtung geführt.

Der Dentalsensor 107 kann an einem elastischen Streifen 121 befestigt sein, der zwischen dem ersten und/oder dem zweiten Nutabschnitt 117-1, 117-2 gehalten wird. Durch eine Miniaturisierung des Dentalsensors 107 kann ein Tragekomfort erhöht werden. Der Dentalsensor 107 ist beispielsweise als ein dünner Patch ausgebildet.

Die elektrischen Leiter 129 können in der Sensorschiene 105 integriert sein und sich über den gesamten Bogen erstrecken, oder mittels des elastischen Streifens 121 zur Verfügung gestellt werden. Der Dentalsensor 107 umfasst zwei Kontaktflächen 135, die an dem jeweiligen elektrischen Leiter 129 anliegen. Dadurch wird der Dentalsensor 107 mit elektrischer Leistung zum Durchführen der Messung versorgt. Eine Elektronik 137 dient zum Erfassen von Messdaten. Die Elektronik 137 und elektrischen Leiter 129 können ebenfalls mit einem additiven Fertigungsverfahren hergestellt werden.

Der Dentalsensor 107 kann eine Antriebsvorrichtung 113, wie beispielsweise einen elektrischen Mikromotor, umfassen, die den Dentalsensor 107 entlang der Sensorschiene 105 fortbewegt. Hierzu kann die Antriebsvorrichtung 113 des Dentalsensors 107 in das Zahnprofil entlang der Sensorschiene 105 eingreifen. Durch die Antriebsvorrichtung 113 kann der Dentalsensor 107 autonom unterschiedliche Positionen an der Sensorschiene 105 anfahren.

Die Sensorschiene 105 kann an der ersten und/oder der zweiten Zahnbefestigungsvorrichtung 101-1 und 101-2 lösbar befestigbar sein. In diesem Fall können zuerst die Zahnbefestigungsvorrichtungen 101-1 und 101-2 ohne die Sensorschiene 105 im Mundraum befestigt werden. Befinden sich diese an der gewünschten Position, wird anschließend die Sensorschiene 105 an diesen befestigt.

Zu diesem Zweck können die Zahnbefestigungsvorrichtungen 101-1 und 101-2 einen Steck- oder Klickmechanismus als Verbindungselement umfassen. Der Steck- oder Klickmechanismus kann durch eine Schienenrasterung und/oder ein Spreizelement gebildet sein, das an der Sensorschiene 105 oder den Zahnbefestigungsvorrichtungen 101-1 und 101-2 angeordnet ist. Dieses Element kann ein Einschieben der Sensorschiene 105 ermöglichen und einen sicheren Halt gewährleisten und in einer gegenüberliegenden Vorrichtung eingesetzt oder eingeklickt werden.

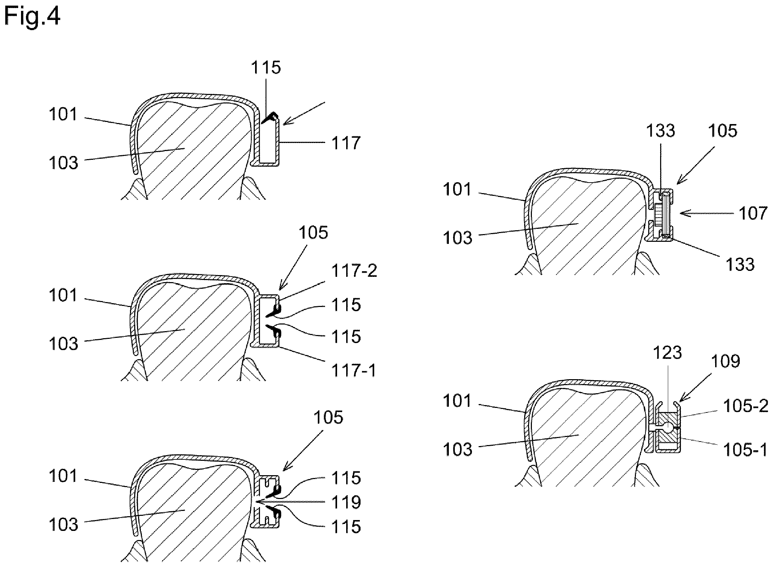

Fig. 4 zeigt unterschiedliche Ausführungsformen der Sensorschiene 105. Die Sensorschiene 105 kann taschenförmig nach oben offen sein, so dass der Dentalsensor 107 von oben eingesetzt werden kann (oben). Die Sensorschiene 105 kann aber auch einen obere und eine unteren Nutabschnitt 117-1 und 117-2 umfassen, von denen der Dentalsensor 107 gehalten wird (Mitte).

Daneben kann die Sensorschiene 105 auch eine Durchgangsöffnung 119 zum Gebiss hin aufweisen, durch die eine direkte Messung, insbesondere des Speichelflusses, an den Zähnen 103 und/oder Zahnfleisches durch den Dentalsensor 107 ermöglicht wird. Die Durchgangsöffnung 119 erstreckt sich beispielsweise über die Länge der Sensorschiene 105, so dass der Dentalsensor 107 an jedem Punkt der Sensorschiene 105 direkte Messungen den Zähnen durchführen kann.

Weitere Ausgestaltungen, die den Speichelfluss unterstützen, wie Speichelflusskanäle, von gingival zu okklusal oder okklusal zu gingival können in der Sensorschiene 105 ebenfalls vorgesehen sein. Zudem kann die Sensorschiene 105 ein Zahnprofil 125 zum Eingreifen des Dentalsensors 107 umfassen. Dadurch kann der Dentalsensor 107 entlang der Sensorschiene 105 stabilisiert werden und ein Verrutschen oder unbeabsichtigtes Bewegen des Dentalsensors 107 verhindert werden.

Die Sensorschiene 105 kann Rillen aufweisen, die elektrische Leiter 129 und Dichtlippen 115 zum Abdecken der elektrischen Leiter 129 umfassen. Die Rillen mit dem elektrischen Leiter 129 können zur Überbrückung einer Spannungsversorgung oder als Datenleitungen dienen. Die elektrischen Leiter 129 werden durch eine Spannungsversorgungsvorrichtung 111 für die Elektronik des Dentalsensors 107 gespeist.

Der Dentalsensor 107 kann eine Antriebsvorrichtung 113, wie beispielsweise einen Mikromotor, zum autonomen Bewegen des Dentalsensors 107 entlang der Sensorschiene 105 umfassen.

Abstandselemente 133 dienen dazu, dass der Dentalsensor 107 entlang der Sensorschiene 105 in einem definierten Abstand zum Zahn 103 angeordnet bleibt (unten). Die Abstandselemente 133 sind durch einen Auflagebereich gebildet, der um die Sensorschiene 105 herumläuft und die Dentalsensoren 107 abstützt.

Eine genaue Positionierung des Dentalsensors 107 kann beispielsweise mittels GPS erfolgen. Denkbar wäre auch, dass dem Benutzer mittels eines Smartphones eine Abbildung der Sensorschiene 105 angezeigt wird und der Dentalsensor 107 auf der Abbildung in der angeordneten Position eingestellt werden kann.

Die erste und/oder zweite Zahnbefestigungsvorrichtung 101-1 und/oder 101-2 kann auch einen Kugelkopf 123 zum Befestigen der Sensorschiene 105 umfassen. Diese Anordnung ermöglicht zudem eine nachträgliche Einstellung der Sensorschiene 105 durch Drehung um die Lagerungspunkte.

Der Kugelkopf 123 bildet ein Verbindungselement, das ein Einklemmen der Sensorbefestigungsvorrichtung 109 ermöglicht, wenn die Positioniervorrichtung zusammengesteckt wird, indem erst die untere Sensorschiene 105 und danach die obere Sensorschiene nach einem Positionieren des Dentalsensors 107 durchgeführt wird.

Die Sensorschienen 105 sind nicht zwingend elastisch ausgestaltet. Dadurch kann der Dentalsensor 107 zwischen den Sensorschienen 105 an eine gewünschte Position eingebracht werden.

Der Dentalsensor 107 kann eine elastische Hülle aufweisen und somit in einer eher starren Sensorschiene 105 positioniert werden. Die Sensorschiene 105 kann ein Endstück umfassen und der Dentalsensor 107 kann seitlich in die Sensorschiene hineingeschoben werden. Die obere und die untere Sensorschiene 107 sind beispielsweise an beiden oder an einem Ende miteinander verbunden. Die Schienenstränge könnte einteilig hergestellt werden und über ein oder zwei aufsteckbares Endstück verbunden werden. An den Endstücken können negativ ausgeprägte Geometrien vorgesehen sein, die mit einem Verbindungselement, wie beispielsweise einem Kugelkopf, verbunden werden können. Es sind jedoch auch unterschiedliche Verbindungselemente denkbar.

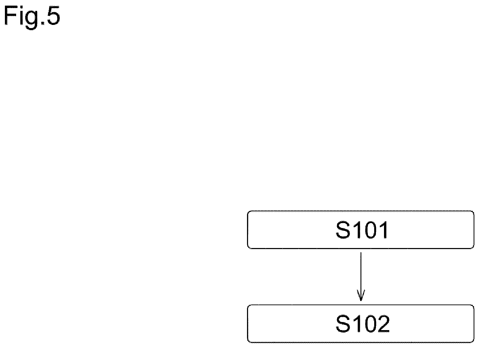

Fig. 5 zeigt ein Blockdiagramm eines Verfahrens zum Positionieren eines Dentalsensors 107. Das Verfahren umfasst den Schritt S101 eines Befestigens der dentalen Sensorbefestigungsvorrichtung 109 mit der ersten Zahnbefestigungsvorrichtung 101-1 an dem ersten Zahn 103-1 und der zweiten Zahnbefestigungsvorrichtung 101-2 an dem zweiten Zahn 103-2. In Schritt S102 wird der Dentalsensor 107 an der Sensorschiene 105 zwischen der ersten Zahnbefestigungsvorrichtung 101-1 und der zweiten Zahnbefestigungsvorrichtung 101-2 befestigt und eine Messung durch den Dentalsensors 107 durchgeführt.

Durch das dentale Sensorsystem 100 entstehen höhere Freiheitsgrade bei der Messung. Soll an mehreren Messpunkten gemessen werden, lässt sich die Anzahl an Dentalsensoren verringern, da mit einem einzigen Dentalsensor an unterschiedlichen Messpunkten gemessen werden kann. Es kann eine individuelle Sensorschiene 105 verwendet werden, mit einer eine labiale oder linguale Messmöglichkeit geschaffen wird. Es wird eine abnehmbare Messvorrichtung realisiert werden, bei der Einzelteile leicht ersetzt oder angepasst werden können. Insgesamt bildet das dentale Sensorsystem 100 eine leicht anbringbare und verschlucksichere, dentale Fixierung von Dentalsensoren 107.

Einzelne Dentalsensoren 107 können mit der Zunge aktiviert und deaktiviert werden, indem mit der Zunge auf den jeweiligen Dentalsensor 107 gedrückt wird. Die Aktivierung und Deaktivierung des Dentalsensors 107 kann auch mittels eines Smartphones erfolgen.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentales Sensorsystem
- 101: Zahnbefestigungsvorrichtung
- 103: Zahn
- 105: Sensorschiene
- 107: Dentalsensor
- 109: Sensorbefestigungsvorrichtung
- 111: Spannungsversorgungsvorrichtung
- 113: Antriebsvorrichtung
- 115: Dichtlippe
- 117: Nutabschnitt
- 119: Durchgangsöffnung
- 121: elastischer Streifen
- 123: Kugelkopf
- 125: Zahnprofil
- 127: Bügel
- 129: elektrischer Leiter
- 131: Datenübermittlungsvorrichtung
- 133: Abstandselemente
- 135: Kontaktflächen
- 137: Elektronik

## Patentansprüche

1. Dentales Sensorsystem (100), mit
- einer ersten Zahnbefestigungsvorrichtung (101-1) zum Befestigen an einem ersten Zahn (103-1);
- einer zweiten Zahnbefestigungsvorrichtung (101-2) zum Befestigen an einem zweiten Zahn (103-2);
- einer Sensorschiene (105) zwischen der ersten und der zweiten Zahnbefestigungsvorrichtung (101-1; 101-2); und
- mindestens einem Dentalsensor (107), der entlang der Sensorschiene (105) positionierbar ist.

2. Dentales Sensorsystem (100) nach Anspruch 1, wobei die erste und/oder zweite Zahnbefestigungsvorrichtung (101-1, 101-2) durch einen Zahnaufsatz gebildet sind.

3. Dentales Sensorsystem (100) nach einem der vorangehenden Ansprüche, wobei der mindestens eine Dentalsensor (107) entlang der Sensorschiene (105) verschiebbar ist.

4. Dentales Sensorsystem (100) nach einem der vorangehenden Ansprüche, wobei die Sensorschiene (105) an der ersten und/oder der zweiten Zahnbefestigungsvorrichtung (101-1, 101-2) befestigbar ist.

5. Dentales Sensorsystem (100) nach Anspruch 4, wobei die erste und/oder zweite Zahnbefestigungsvorrichtung (101-1, 101-2) einen Kugelkopf (123) zum Befestigen der Sensorschiene (105) umfasst.

6. Dentales Sensorsystem (100) nach einem der vorangehenden Ansprüche, wobei der Dentalsensor (107) auf/in die Sensorschiene (105) aufsetzbar oder aufklickbar ist.

7. Dentales Sensorsystem (100) nach einem der vorangehenden Ansprüche, wobei die Sensorschiene (105) einen ersten Nutabschnitt (117-1) und/oder einen zweiten Nutabschnitt (117-2) zum Führen des Dentalsensors (107) umfasst.

8. Dentales Sensorsystem (100) nach Anspruch 7, wobei der Dentalsensor (107) einen elastischen Streifen (121) zum Einsetzen zwischen dem ersten und/oder dem zweiten Nutabschnitt (117-1, 117-2) umfasst.

9. Dentales Sensorsystem (100) nach einem der vorangehenden Ansprüche, wobei die Sensorschiene (105) eine Durchgangsöffnung (119) zum Gebiss hin und/oder ein Zahnprofil (125) zum Eingreifen des Dentalsensors (107) umfasst.

10. Dentales Sensorsystem (100) nach einem der vorangehenden Ansprüche, wobei der Dentalsensor (107) eine Antriebsvorrichtung (113) zum Bewegen des Dentalsensors (107) entlang der Sensorschiene (105) umfasst.

11. Dentales Sensorsystem (100) nach einem der vorangehenden Ansprüche, wobei die das Sensorsystem (100) eine Spannungsversorgungsvorrichtung (111) und/oder eine Datenübermittlungsvorrichtung (131) für den Dentalsensor (107) umfasst

12. Dentale Sensorbefestigungsvorrichtung (109), mit:
- einer ersten Zahnbefestigungsvorrichtung (101-1) zum Befestigen an einem ersten Zahn (103-1);
- einer zweiten Zahnbefestigungsvorrichtung (101-2) zum Befestigen an einem zweiten Zahn (103-2);
- einer Sensorschiene (105) zwischen der ersten und der zweiten Zahnbefestigungsvorrichtung (101-1; 101-2), auf der ein Dentalsensor (107) positionierbar ist.

13. Dentalsensor (107) für eine dentale Sensorbefestigungsvorrichtung (109) nach Anspruch 12.

14. Verfahren zum Positionieren eines Dentalsensors (107), mit den Schritten:
- Befestigen (S101) einer dentalen Sensorbefestigungsvorrichtung (109) mit einer ersten Zahnbefestigungsvorrichtung (101-1) an einem ersten Zahn (103-1) und einer zweiten Zahnbefestigungsvorrichtung (101-2) an einem zweiten Zahn (103-2); und
- Positionieren (S102) eines Dentalsensors (107) an einer Sensorschiene (105) zwischen der ersten Zahnbefestigungsvorrichtung (101-1) und der zweiten Zahnbefestigungsvorrichtung (101-2).

15. Verwenden einer Sensorschiene (105) zwischen einer ersten Zahnbefestigungsvorrichtung (101-1) und einer zweiten Zahnbefestigungsvorrichtung (101-2) zum Positionieren eines Dentalsensors (107).
